(19) Europäisches Patentamt
European Patent Office
Office européen des brevets

(11) Publication number: 0 129 321
B1

(12) # EUROPEAN PATENT SPECIFICATION

(45) Date of publication of patent specification: 15.03.89

(21) Application number: 84303225.1

(22) Date of filing: 11.05.84

(51) Int. Cl.[4]: C 07 D 295/08,
C 07 D 265/30,
C 07 D 211/14, A 01 N 33/08
// C07D303/04

(54) **4-Phenyl-2-hydroxybutyl piperidines or morpholines as fungicides.**

(30) Priority: 16.06.83 GB 8316444

(43) Date of publication of application:
27.12.84 Bulletin 84/52

(45) Publication of the grant of the patent:
15.03.89 Bulletin 89/11

(84) Designated Contracting States:
BE CH DE FR GB IT LI NL SE

(56) References cited:
DE-A-2 800 755
FR-M- 5 012
US-A-4 045 568

(73) Proprietor: IMPERIAL CHEMICAL INDUSTRIES PLC
Imperial Chemical House Millbank
London SW1P 3JF (GB)

(72) Inventor: Worthington, Paul Anthony
4 Oakhurst Road Maidenhead Court Park
Maidenhead Berkshire (GB)
Inventor: DeFraine, Paul
5 Salisbury Close
Wokingham Berkshire (GB)

(74) Representative: Alner, Henry Giveen Hamilton et al
Imperial Chemical Industries PLC Legal Department: Patents P.O. Box 6 Bessemer Road
Welwyn Garden City Herts, AL7 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 129 321 B1

Courier Press, Leamington Spa, England.

**Description**

This invention relates to morpholine and piperidine compounds useful as fungicides, to a process for preparing them, to fungicidal compositions containing them, and to methods of using them to combat fungi, especially fungal infections in plants.

French Medicament Patent FR—M—5012 (A) describes a very wide range of amine derivatives having a plurality of pharmacological properties, none of which is anti-mycotic in character. Furthermore these derivatives are all ethers and there is not mention of any of them possessing plant fungicidal activity.

The invention provides compounds having the general formula (I):

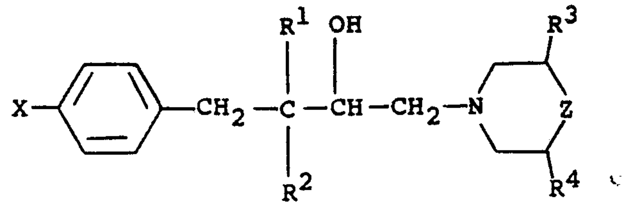

(I)

and stereoisomers, and acid addition salts, thereof, wherein $R^1$, $R^2$, $R^3$, and $R^4$ each represent a hydrogen atom or an alkyl group containing from 1 to 4 carbon atoms, X represents a hydrogen or a halogen atom, or an alkyl, cyclopropyl, cyclobutyl, cyclopentyl, cyclohexyl, alkenyl containing up to 6 carbon atoms, alkynyl containing up to 6 carbon atoms, or a $C_{1-6}$ alkoxy group, and Z represents a methylene group or an oxygen atom; and ester derivatives of the alcohol function which are acetyl, propionyl or benzoyl; and ether derivatives of the alcohol function which are methyl, (except when X is hydrogen or chlorine and $R^1$, $R^2$, $R^3$ and $R^4$ are all hydrogen) ethyl, propyl, butyl or benzyl and their acid addition salts.

The compounds have fungicidal activity.

The compounds of the invention contain at least one chiral centre. Such compounds are generally obtained in the form of isomeric mixtures. However, these and other mixtures can be separated into the individual isomers by methods known in the art.

The alkyl and alkoxy groups may be straight or branched chain groups having 1 to 6, e.g. 1 to 4, carbon atoms; examples are methyl, ethyl, propyl (*n*- or *iso*-propyl) and butyl (*n*-, *sec*-, *iso* or *t*- butyl). Cycloalkyl groups are cyclopropyl, cyclobutyl, cyclopentyl or cychlohexyl.

The alkenyl and alkynyl groups may contain up to 6 carbon atoms.

The salts can be salts with inorganic or organic acids e.g. hydrochloric, nitric, sulphuric, acetic, 4-toluene-sulphonic or oxalic acid.

The ester derivatives of the alcohol are acetyl, propionyl or benzoyl; the ether derivatives are a methyl, ethyl, propyl, butyl or benzyl derivative.

Examples of the compounds of the invention are shown in Table 1. These conform to formula I.

TABLE 1

| COMPOUND NO | X | Z | $R^1$ | $R^2$ | $R^3$ | $R^4$ | Melting point (°C) | Additional comments |
|---|---|---|---|---|---|---|---|---|
| 1 | $t$-$C_4H_9$ | O | $CH_3$ | H | $CH_3$ | $CH_3$ | Oil | Mixture |
| 2 | $t$-$C_4H_9$ | $CH_2$ | $CH_3$ | H | $CH_3$ | $CH_3$ | Oil | Mixture |
| 3 | F | O | $CH_3$ | H | $CH_3$ | $CH_3$ | Oil | - |
| 4 | Cl | O | $CH_3$ | H | $CH_3$ | $CH_3$ | Oil | - |
| 5 | $t$-$C_4H_9$ | $CH_2$ | $CH_3$ | H | H | H | Oil | Mixture |
| 6 | H | $CH_2$ | H | H | H | H | 39-40 | Single isomer |
| 7 | H | O | H | H | H | H | Oil | Single isomer |
| 8 | H | O | H | H | $CH_3$ | $CH_3$ | Oil | Mixture |
| 9 | $t$-$C_4H_9$ | $CH_2$ | $CH_3$ | $CH_3$ | H | H | 81 | Single isomer |
| 10 | $t$-$C_4H_9$ | $CH_2$ | H | H | H | H | Oil | Single isomer |
| 11 | $t$-$C_4H_9$ | O | H | H | $CH_3$ | $CH_3$ | Oil | Mixture |
| 12 | $t$-$C_4H_9$ | $CH_2$ | H | H | $CH_3$ | $CH_3$ | Oil | Mixture |

TABLE 1 (cont)

| COMPOUND NO | X | Z | $R^1$ | $R^2$ | $R^3$ | $R^4$ | Melting point (°C) | Additional comments |
|---|---|---|---|---|---|---|---|---|
| 13 | $t$-$C_4H_9$ | O | $CH_3$ | H | $CH_3$ | $CH_3$ | Oil | O-Acetate of Compound 1 |
| 14 | H | O | H | H | $CH_3$ | $CH_3$ | Oil | O-Acetate of Compound 8 |
| 15 | H | O | H | H | H | H | Oil | O-Acetate of Compound 7 |
| 16 | H | $CH_2$ | H | H | H | H | Oil | O-Acetate of Compound 6 |
| 17 | $t$-$C_4H_9$ | $CH_2$ | $CH_3$ | H | $CH_3$ | $CH_3$ | Oil | Cis $CH_3/CH_3$ Isomer A |
| 18 | $t$-$C_4H_9$ | $CH_2$ | $CH_3$ | H | $CH_3$ | $CH_3$ | Oil | Cis $CH_3/CH_3$ Isomer B |
| 19 | $t$-$C_4H_9$ | $CH_2$ | $CH_3$ | H | $CH_3$ | $CH_3$ | Oil | trans $CH_3/CH_3$ |

TABLE 1 (cont)

| COMPOUND NO | X | Z | $R^1$ | $R^2$ | $R^3$ | $R^4$ | Melting point (°C) | Additional comments |
|---|---|---|---|---|---|---|---|---|
| 20 | $t$-$C_4H_9$ | $CH_2$ | $CH_3$ | H | H | H | 67-8° | Single isomer |
| 21 | $t$-$C_4H_9$ | $CH_2$ | $CH_3$ | H | H | H | Oil | O-Methyl Ether of Compound 20 |
| 22 | $t$-$C_4H_9$ | $CH_2$ | $CH_3$ | H | H | H | Oil | O-Acetate of Compound 20 |

Compounds of general formula (I):

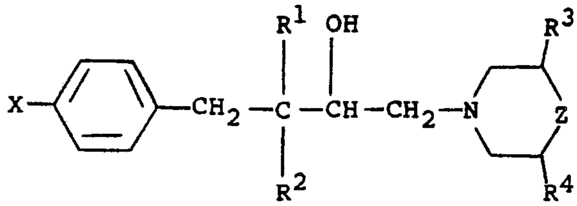

(I)

can be prepared by treatment of an epoxide of general formula (II):

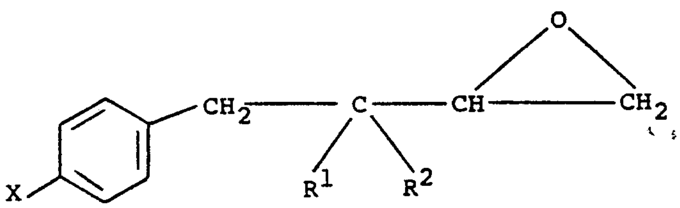

(II)

wherein X, $R^1$, and $R^2$ are as defined above, with a compound of general formula (III):

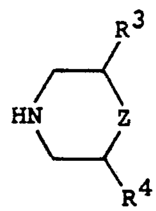

(III)

wherein $R^3$ and $R^4$ are as defined above, in the presence of a convenient solvent such as ethanol or tetrahydrofuran, or, preferably, in the absence of a solvent at a temperature of 20°-100°C.

The epoxides (II) can be prepared by treating an aldehyde of general formula (IV):

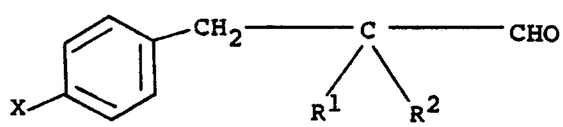

(IV)

with dimethyl oxosulphonium methylide (Corey and Chaykovsky, *J. Amer. Chem. Soc,* 1965, *87,* 1353) or dimethyl sulphonium methylide (Corey and Chaykovsky, J. Amer. Chem. Soc., 1962, *84,* 3782) using methods set out in the literature.

The aldehydes of general formula (IV) can be prepared by methods set out in the literature.

The esters and ethers can be prepared from the parent alcohol compound (I) by successive treatment with a base (preferably sodium hydride) and a suitable alkyl or acyl halide (preferably chloride, bromide, or iodide) in a suitable solvent such as dimethyl formamide or tetrahydrofuran.

The salts of the compounds of the general formula (I) can be prepared from the latter by known methods.

The compounds and salts are active fungicides, particularly against the diseases:

*Piricularia oryzeae* on rice

*Puccinia recondita, Puccinia striiformis* and other rusts on wheat, *Puccinia hordei, Puccinia striiformis* and other rusts on barley, and rusts on other hosts e.g. coffee, apples, vegetables and ornamental plants

*Erysiphe graminis* (powdery mildew) on barley and wheat and other powdery mildews on various hosts such as *Sphaerotheca fuliginea* on cucurbits (e.g. cucumber), *Podosphaera leucotricha* on apples and *Uncinula necator* on vines

*Helminthosporium* ssp., *Rhynchosporium* ssp. and *Pseudocercosporella herpotrichoides* on cereals

*Cercospora arachidicola* on peanuts and other *Cercospora* species on for example sugar beet, bananas and soya beans

*Botrytis cinerea* (grey mould) on tomatoes, strawberries, vines and other hosts

*Venturia inaequalis* (scab) on apples and *Plasmopara viticola* (downy mildew) on vines.

Some of the compounds have also shown a broad range of activities against fungi *in vitro*. They have activity against various post-harvest diseases on fruit (e.g. *Pencillium digatatum* and *italicum* on oranges and *Gloeosporium musarum* on bananas). Further some of the compounds are active as seed dressings

against: *Fusarium* spp., *Septoria* spp., *Tilletia* ssp. (i.e. bunt, a seed borne disease of wheat), *Ustilago* spp., *Helminthosporium* spp. and *Pseudocercosporella herpotrichoides* on cereals, *Rhizoctonia solani* on cotton and *Corticium sasakii* on rice.

The compounds can move acropetally in the plant tissue. Moreover, the compounds can be volatile enough to be active in the vapour phase against fungi on the plant.

They may also be useful as industrial (as opposed to agricultural) fungicides, e.g. in the prevention of fungal attack on wood, hides, leather and especially paint films.

The compounds are also useful for the treatment of candidiasis and human dermatophyte infections.

The compounds may be used as such for fungicidal purposes but are more conveniently formulated into compositions for such usage. The invention thus provides a fungicidal or plant growth regulating composition comprising a compound of general formula (I) as hereinbefore defined, or a salt, ether or ester thereof; and, optionally, a carrier or diluent.

The invention also provides a method of combating fungi, which comprises applying to a plant, to seed of a plant, or to the locus of the plant or seed, a compound, or salt, ether or ester thereof, as hereinbefore defined.

The compounds, salts, ethers and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of compositon used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earch, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg nigrogen-, potassium- or phosphorus-containing fertilisers). Compositions comprising only granules of fertilisers incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or

more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10—85%, for example 25—60%, by weight of the active ingredient(s). These concentrates suitably contain organic acids (eg. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% of active ingredient(s) may be used.

The compositions of this invention can comprise also other compound(s) having biological activity, eg. componds having similar or complementary fungicidal or plant growth activity or compounds having plant growth regulating, herbicidal or insecticidal activity.

The other fungicidal compound can be, for example, one which is capable of combating ear diseases of cereals (eg. wheat) such as *Septoria, Gibberella* and *Helminthosporium* spp., seed and soil borne diseases and downy and powdery mildews on grapes and powdery mildew and scab on apple etc.

These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, fosetyl-aluminium, carboxin, oxycarboxin, fenarimol, nuarimol, fenfuram, methfuroxam, nitrothal-isopropyl, triadimefon, thiabendazole, etridiazole, triadimenol, biloxazol, dithianon, binapacryl, quinomethionate, guazatine, dodine, fentin acetate, fentin hydroxide, dinocap, folpet, dichlofluanid, ditalimfos, kitazin, cycloheximide, diclobutrazol, a dithiocarbamate, a copper compound, a mercury compound, 1-(2-cyano-2-methoxyiminoacetyl)-3-ethyl urea, fenapanil, ofurace, propiconazole, etaconazole and fenpropemorph.

The compounds of general formula (I) can be mixed with soil, peat or other rooting media for the protection of plants against seed-borne, soil-borne or foliar fungal diseases.

Suitable insecticides are pirimicarb, dimethoate, demeton-s-methyl and formothion.

The other plant growth regulating compound can be one which controls weeds or seedhead formation, improves the level or longevity of the plant growth regulating activity of the compounds of general formula (I), selectively controls the growth of the less desirable plants (eg. grasses) or causes the compound of general formula (I) to act faster or slower as a plant growth regulating agent. Some of these other agents will be herbicides.

Examples of suitable plant growth regulating compounds, which can display synergy in admixture, or use, with the invention compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, paclobutrazol, flurprimidol, fluoridamid, mefluidide, substituted quaternary ammonium and phosphonium compounds (eg. chlormequat* chlorphonium or mepiquatchloride), ethephon, carbetamide, methyl-3,6-dichloroanisate, daminozide*, asulam, abscisic acid, isolpyrimol, 1-(4-chlorophenyl)-4,6-dimethyl-2-oxo-1,2-dihydropyridine-3-carboxylic acid, hydroxoybenzonitriles (eg. bromoxynil), difenzoquat, benzoylprop-ethyl 3,6-dichloropicolinic acid, and tecnazene. Synergy will be most likely to occur with those of the foregoing which are quaternary ammonium compounds in particular those marked with an asterisk.

The following Examples illustrate the invention; the temperatures are given in degrees Centrigrade (°C).

Example 1

This Example illustrates the preparation of 1-[4-(p-tert.butyl-phenyl)-3-methyl-2-hydroxy-butyl]-3,5-dimethyl-piperidine. (Compound no 2 of Table 1).

A mixture of 3-(p-tert.butyl-phenyl)-2-methyl-propion-aldehyde (0.02 mol, 4.08 g), trimethyl sulphonium iodide (0.02 mol, 4.08 g), and potassium hydroxide (2.24 g) in acetonitrile (40 ml) and water (0.1 ml) were stirred at 60°C for 2 hours. After cooling to room temperature, the solution was filtered and the acetonitrile removed in vacuo. The residue was extracted with diethyl ether (150 ml); the etheral extracts washed with water (4×100 ml), and dried over anhydrous sodium sulphate. Removal of the solvent gave 4-(p-tert.butyl-phenyl)-3-methyl-1,2-epoxy-butane (90%) as a pale yellow oil.

A solution of 4-(p-tert.butyl-phenyl)-3-methyl-1,2-epoxy-butane (2.5 g) and 3,5-dimethyl-piperidine (2.5 g) were refluxed in tetrahydrofuran (10 ml) for 8 hours. The reaction mixture was poured into water (50 ml) and extracted with diethyl ether (2×75 ml). The ethereal extracts were washed with water (3×100 ml) and dried over anhydrous sodium sulphate. Removal of the solvent gave a yellow oil which was dissolved in diethyl ether (5 ml) and ethereal hydrochloric acid added to give a white crystalline solid. This was filtered off, neutralised with saturated sodium bicarbonate solution, and extracted with diethyl ether. The ethereal extracts were washed with water, dried over anhydrous sodium sulphate, and evaporated to give the title compound (0.5 g) as a yellow oil.

Example 2

This Example illustrates the preparation of 1-[4-(p-tert.butyl-phenyl)-3-methyl-2-hydroxy-butyl]-piperidine, (Compound No. 5 in Table 1) and its separation into a single diastereomer. (Compound No. 20 in Table 1).

A solution of 4-(p-tert.butyl-phenyl)-3-methyl-1,2-epoxy-butane (11.0 g) and piperidine (11.0 g) were refluxed in tetrahydrofuran (50 ml) for 10 hours. The reaction mixture was cooled, poured into water (100 ml) and extracted with diethyl ether (2×100 ml). The ethereal extracts were washed with water (3×100 ml) and dried over anhydrous sodium sulphate. Removal of the solvent gave a yellow oil which was purified by flash column chromatography (Crosfield silica SD210 eluted with ethyl acetate/methanol 9:1) to give the title compound (No. 5 in Table I) (2.1 g) as a pale yellow oil. This exists as a mixture of diastereomer in a ratio of 1:1.

Separation of the diastereomers.

A mixture of the diastereomers in a ratio of 1:1 was partially separated by eluting batches (10 g) down a Waters Preparative 500, liquid chromatography machine using hexane (60—80°, 92%)/acetone (8%) as the eluant with one Prepak-500/silica column at a flow rate of 0.2l/minute. The last quarter tail end of the peak was cut off to give a product with a diastereomeric ratio of 3:1. A sample (7 g) of this partially separated product, was dissolved in warm petroleum ether (60—80°, 40 ml) and cooled in an acetone-dry ice bath. The title compound (No. 20 in Table I) is a white crystalline solid (3.2 g, mp. 67—8°) was filtered off, this was shown to the the pure diastereomer (96%) by capillary gas chromatography and carbon 13 nmr spectroscopy.

Example 3

An emulsifiable concentrate was made up by mixing the ingredients, and stirring the mixture until all the constituents were dissolved.

| | |
|---|---|
| Compound of Example 1 | 10% |
| Ethylene dichloride | 40% |
| Calcium dodecylbenzenesulphate | 5% |
| "Lubrol" L | 10% |
| "Aromasol" H | 35% |

(The words "LUBROL" and "AROMASOL" are Registered Trade Marks)

Example 4

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44—100, to obtain the desired size of grains.

| | |
|---|---|
| Compound of Example 2 | 50% |
| "Dispersol" T | 25% |
| "Lubrol"APN5 | 1.5% |
| Sodium acetate | 23.5% |

(The words "DISPERSOL" and "LUBROL" are Registered Trade Marks)

Example 5

The ingredients were all ground together to produce a powder formulation readily dispersible in liquids.

| | |
|---|---|
| Compound of Example 1 | 45% |
| "Dispersol" T | 5% |
| "Lissapol" NX | 0.5% |
| "Cellofas" B600 | 2% |
| Sodium acetate | 47.5% |

(The words "DISPERSOL", "LISSAPOL" and "CELLOFAS" are Registered Trade Marks)

Example 6

The active ingredient was dissolved in a solvent and the resultant liquid was sprayed on to the granules of China clay. The solvent was then allowed to evaporate to produce a granular composition.

| | |
|---|---|
| Compound of Example 2 | 5% |
| China clay granules | 95% |

Example 7

A composition suitable for use as a seed dressing was prepared by mixing the three ingredients.

| | |
|---|---|
| Compound of Example 1 | 50% |
| Mineral oil | 2% |
| China clay | 48% |

Example 8

A dusting powder was prepared by mixing the active ingredient with talc.

| | |
|---|---|
| Compound of Example 2 | 5% |
| Talc | 95% |

Example 9

A Col formulation was prepared by ball-milling the constituents set out below and then forming an aqueous suspension of the ground mixture with water.

| | |
|---|---|
| Compound of Example 1 | 40% |
| "Dispersol" T | 10% |
| "Lubrol" APN5 | 1% |
| Water | |

(The words 'DISPERSOL" and "LUBROL" are Registered Trade Marks)

A dispersible powder formulation was made by mixing together the ingredients set out below and then grinding the mixture until all were thoroughly mixed.

Example 10

| | |
|---|---|
| Compound of Example 2 | 25% |
| "Aerosol" OT/B | 2% |
| "Dispersol" A.C. | 5% |
| China clay | 28% |
| Silica | 40% |

(The words "AEROSOL" and "DISPERSOL" are Registered Trade Marks)

Example 11

This Example illustrates the preparation of a dispersible powder formulation. The ingredients were mixed and the mixture then ground in a comminution mill.

| | |
|---|---|
| Compound of Example 1 | 25% |
| "Perminal" BX | 1% |
| "Dispersol" T | 5% |
| Polyvinylpyrrolidone | 10% |
| Silica | 25% |
| China clay | 34% |

(The words "PERMINAL" and "DISPERSOL" are Registered Trade Marks)

Example 12

The ingredients set out below were formulated into a dispersible powder by mixing then grinding the ingredients.

| | |
|---|---|
| Compound of Example 2 | 25% |
| "Aerosol" OT/B | 2% |
| "Dispersol" A | 5% |
| China clay | 68% |

(The words "AEROSOL" and "DISPERSOL" are Registered Trade Marks)

In Examples 3 to 12 the proportions of the ingredients given are by weight.

The compounds set out in Table 1 and numbered 3 to 22 were similarly formulated as specifically described in Examples 3 to 12.

There now follows an explanation of the compositions or substances represented by the various Trade Marks and Trade Names mentioned above.

| | |
|---|---|
| LUBROL L: | a condensate of nonyl phenol 1 mole) with ethylene oxide (13 moles) |
| AROMASOL H: | a solvent mixture of alkylbenzenes |
| DISPERSOL T & AC: | a mixture of sodium sulphate and a condensate of formaldehyde with sodium naphthalene sulphonate |
| LUBROL APN5: | a condensate of nonyl phenol (1 mole) with naphthalene oxide (5.5 moles) |
| CELLOFAS B600: | a sodium carboxymethyl cellulose thickener |
| LISSAPOL NX: | a condensate of nonyl phenol (1 mole) with ethylene oxide (8 moles) |
| AEROSOL OT/B: | dioctyl sodium sulphosuccinate |
| PERMINAL BX: | a sodium alkyl naphthalene sulphonate |

Example 13

The compounds were tested against a variety of foliar fungal diseases of plants. The technique employed was as follows.

The plants were grown in John Innes Potting Compost (No 1 or 2) in 4 cm diameter minipots. The test compounds were formulated either by bead milling with aqueous Dispersol T (the word "DISPERSOL" is a Registered Trade Mark) or as a solution in acetone or acetone/ethanol which was diluted to the required concentration immediately before use. For the foliage diseases, the formulations (100 ppm active ingredient) were sprayed on to the foliage and applied to the roots of the plants in the soil. The sprays were applied to maximum retention and the root drenches to a final concentration equivalent to approximately 40 ppm a.i./dry soil. Tween 20, (the word "TWEEN" is a Registered Trade Mark) to give a final concentration of 0.05%, was added when the sprays were applied to cereals.

For most of the tests the compound was applied to the soil (roots) and to the foliage (by spraying) one or two days before the plant was inoculated with the disease. An exception was the test on *Erysiphe graminis* in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading:—

4 = no disease
3 = trace — 5% of disease on untreated plants
2 = 6—25% of disease on untreated plants
1 = 26—59% of disease on untreated plants
0 = 60—100% of disease on untreated plants

The results are shown in Table II.

TABLE II

| COMPOUND NUMBER | PUCCINIA RECONDITA (WHEAT) | ERYSIPHE GRAMINIS (BARLEY) | CERCOSPORA ARACHIDICOLA (PEANUT) | VENTURIA INAEQUALIS (APPLE) |
|---|---|---|---|---|
| 1 | 0 | 4 | 0 | 0 |
| 2 | 4 | 4 | 3 | 0 |
| 3 | 0 | 3 | 0 | 0 |
| 4 | 0 | 3 | 0 | 0 |
| 5 | 4 | 4 | 0 | 4 |
| 6 | 2 | 0 | 1 | 0 |
| 9 | 0 | 4 | 1 | 0 |
| 10 | 4 | 4 | 2 | 0 |

"-" means compound not tested.

TABLE II

| COMPOUND NUMBER | PUCCINIA RECONDITA (WHEAT) | ERYSIPHE GRAMINIS (BARLEY) | CERCOSPORA ARACHIDICOLA (PEANUT) | VENTURIA INAEQUALIS (APPLE) |
|---|---|---|---|---|
| 11 | 0 | 4 | 3 | 2 |
| 12 | 0 | 4 | 2 | 2 |
| 13 | 4 | 4 | 0 | 0 |
| 14 | 0 | 0 | 0 | 4 |
| 15 | 0 | 2 | 0 | 0 |
| 17 | 0 | 4 | 0 | - |
| 18 | 0 | 4 | 4 | 0 |
| 19 | 3 | 4 | 3 | 0 |
| 20 | 3 | 4 | 3 | 0 |
| 21 | 0 | 4 | 0 | 0 |
| 22 | - | - | - | - |

"-" means compound not tested.

**Claims**

1. A compound having the general formula (I):

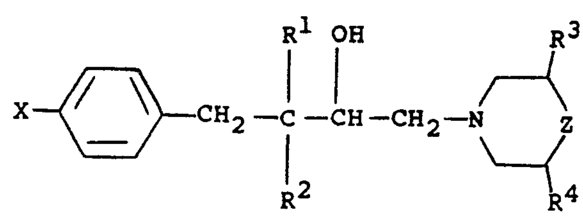

(I)

and stereoisomers, and acid addition salts, thereof, wherein $R^1$, $R^2$, $R^3$ and $R^4$ each represent a hydrogen atom or an alkyl group containing from 1 to 4 carbon atoms; X represents a hydrogen or a halogen atom, or a $C_{1-6}$ alkyl, cyclopropyl, cyclobutyl, cyclopentyl, cyclohexyl, alkenyl containing up to 6 carbon atoms, alkynyl containing up to 6 carbon atoms, or a $C_{1-6}$ alkoxy group; and Z represents a methylene group or an oxygen atom; and ester derivatives of the alcohol function which are acetyl, propionyl or benzoyl; and ether derivatives of the alcohol function which are methyl, (except when X is hydrogen or chlorine and $R^1$, $R^2$, $R^3$ and $R^4$ are all hydrogen) ethyl, propyl, butyl or benzyl; and their acid addition salts.

2. A compound as claimed in Claim 1 wherein $R^1$, $R^2$, $R^3$ and $R^4$ are each hydrogen or methyl.

3. A compound as claimed in Claim 1 or Claim 2 wherein X is hydrogen, chlorine or fluorine or methyl, ethyl, propyl (*n-* or *iso-*propyl) or butyl (*n-*, *sec-*, *iso-* or *t-* butyl).

4. A compound as claimed in any of Claims 1 to 3 wherein Z is $-CH_2-$.

5. 1-[4-p-tert.butyl-phenyl)-3-methyl-2-hydroxy-butyl]-3,5-dimethyl-piperidine.

6. 1-[4-p-tert.butyl-phenyl)-3-methyl-2-hydroxy-butyl]-piperidine.

7. The compound of Claim 6 in the form of its isomer having a melting point of 67—68°C.

8. A process for preparing the compounds claimed in claim 1 and formula (I):

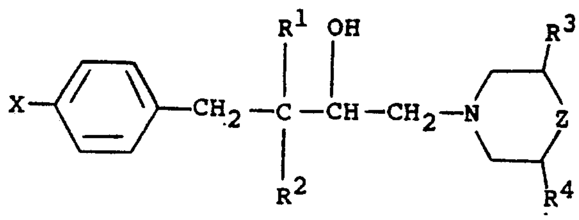

(I)

wherein $R^1$, $R^2$, $R^3$ and $R^4$ each represent a hydrogen atom or an alkyl group containing from 1 to 4 carbon atoms; X represents a hydrogen or a halogen atom, or a $C_{1-6}$ alkyl, cyclopropyl, cyclobutyl, cyclopentyl, cyclohexyl, alkenyl containing up to 6 carbon atoms, alkynyl containing up to 6 carbon atoms, or a $C_{1-6}$ alkoxy group; and Z represents a methylene group or an oxygen atom, which comprises bringing into reaction an epoxide of general formula:

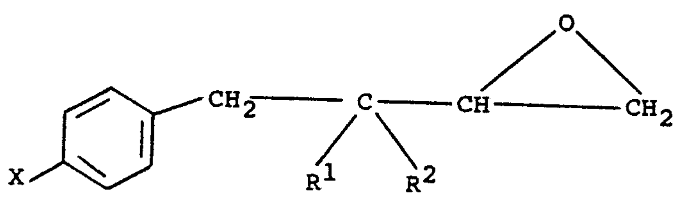

(II)

wherein X, $R^1$ and $R^2$ are as defined above, with a compound of general formula (III):

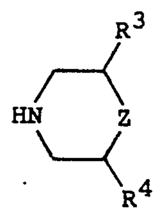

(III)

wherein $R^3$, $R^4$ and Z are as defined above, in the presence of a convenient solvent such as ethanol or tetrahydrofuran, or, preferably, in the absence of a solvent at a temperature of 20—100°C.

9. A process as claimed in Claim 8, wherein the epoxide (II) is prepared by treating an aldehyde of general formula (IV):

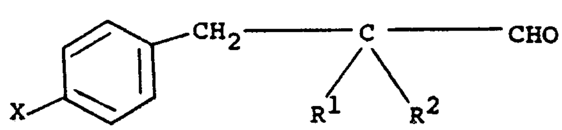

(IV)

with dimethyl oxosulphonium methylide or dimethyl sulphonium methylide.

10. A fungicidal composition comprising a compound having the general formula (I):

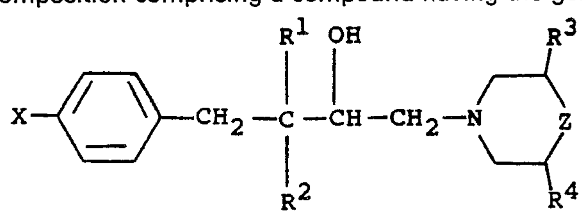

(I)

wherein $R^1$, $R^2$, $R^3$ and $R^4$ each represent a hydrogen atom or an alkyl group containing from 1 to 4 carbon atoms; X represents a hydrogen or a halogen atom, or a $C_{1-6}$ alkyl, cyclopropyl, cyclobutyl, cyclopentyl, cyclohexyl, alkenyl containing up to 6 carbon atoms, alkynyl containing up to 6 carbon atoms, or a $C_{1-6}$ alkoxy group; and Z represents a methylene group or an oxygen atom; or a stereoisomer, or an acid addition salt, thereof; or an ester derivative of the alcohol function which is acetyl, propionyl or benzoyl; or an ether derivative of the alcohol function with is methyl, ethyl, propyl, butyl or benzyl; and a carrier or diluent.

11. A method of combating fungal diseases in a plant, which method comprises applying to the plant, to seed of the plant or to the locus of the plant or seed, a compound having the general formula (I):

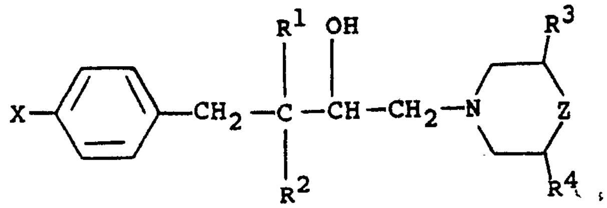

(I)

wherein $R^1$, $R^2$, $R^3$ and $R^4$ each represent a hydrogen atom or an alkyl group containing from 1 to 4 carbon atoms; X represents a hydrogen or a halogen atom, or a $C_{1-6}$ alkyl, cyclopropyl, cyclobutyl, cyclopentyl or cyclohexyl, alkenyl containing up to 6 carbon atoms, alkynyl containing up to 6 carbon atoms, or a $C_{1-6}$ alkoxy group; and Z represents a methylene group or an oxygen atom; or a stereoisomer, or an acid addition salt, thereof; or an ester derivative of the alcohol function which is acetyl, propionyl or benzoyl; or an ether derivative of the alcohol function with is methyl, ethyl, propyl, butyl or benzyl; or a composition as defined in Claim 10.

**Patentansprüche**

1. Verbindungen der allgemeinen Formel (I)

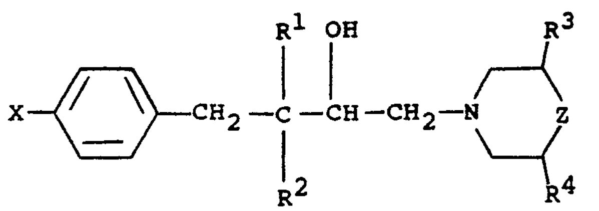

(I)

und Stereoisomere und Säureadditionssalze davon, worin $R^1$, $R^2$, $R^3$ und $R^4$ jeweils für ein Wasserstoffatom oder eine Alkylgruppe mit 1 bis 4 Kohlenstoffatomen stehen, X für ein Wasserstoff- oder Halogenatom oder eine $C_{1-6}$-Alkyl-, Cyclopropyl-, Cyclobutyl-, Cyclopentyl-, Cyclohexyl-, Alkenyl- (mit bis zu 6 Kohlenstoffatomen), Alkinyl- (mit bis zu 6 Kohlenstoffatomen) oder $C_{1-6}$-Alkoxygruppe steht und Z für eine Methylengruppe oder ein Sauserstoffatom steht; und Esterderivate der Alkoholfunktion, welche Acetyl-, Propionyl- oder Benzylderivate sind; und Etherderivate der Alkoholfunktion, welche Methyl (außer wenn X für Wasserstoff oder Chlor steht und $R^1$, $R^2$, $R^3$ und $R^4$ alle für Wasserstoff stehen), Ethyl-, Propyl-, Butyl- oder Benzylderivate sind; und deren Säureadditionssalze.

2. Verbindungen nach Anspruch 1, worin $R^1$, $R^2$, $R^3$ und $R^4$ jeweils für Wasserstoff oder Methyl stehen.

3. Verbindungen nach Anspruch 1 oder 2, worin X für Wasserstoff, Chlor oder Fluor oder Methyl, Ethyl, Propyl (n- oder Isopropyl) oder Butyl (n-, sec-, Iso- oder t-Butyl) steht.

4. Verbindungen nach einem der Ansprüche 1 bis 3, worin Z für $-CH_2-$ steht.

5. 1-[4-(p-tert.-Butyl-phenyl)-3-methyl-2-hydroxy-butyl]-3,5-dimethyl-piperidin.

6. 1-[4-(p-tert.-Butyl-phenyl)-3-methyl-2-hydroxy-butyl]piperidin.

7. Verbindung nach Anspruch 6 in Form des Isomers, welches einen Schmelzpunkt von 67 bis 68°C aufweist.

8. Verfahren zur Herstellung der Verbindungen nach Anspruch 1 und der Formel (I)

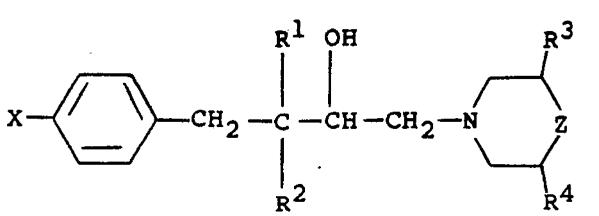

(I)

worin $R^1$, $R^2$, $R^3$ und $R^4$ jeweils für ein Wasserstoffatom oder eine Alkylgruppe mit 1 bis 4 Kohlenstoffatomen stehen, X für ein Wasserstoff- oder Halogenatom oder eine $C_{1-6}$-Alkyl-, Cyclopropyl-, Cyclobutyl-, Cyclopentyl-, Cyclohexyl-, Alkenyl- (mit bis zu 6 Kohlenstoffatomen), Alkinyl- (mit bis zu 6 Kohlenstoffatomen) oder $C_{1-6}$-Alkoxygruppe steht und Z für eine Methylengruppe oder eine Sauerstoffatom steht, bei welchem ein Epoxid der allgemeinen Formel (II)

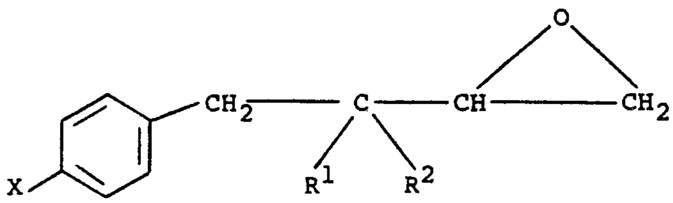

(II)

worin X, $R^1$ und $R^2$ die oben angegebenen Bedeutungen besitzen, mit einer Verbindung der allgemeinen Formel (III)

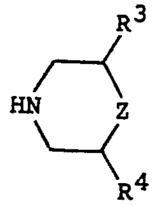

(III)

worin $R^3$, $R^4$ und Z die oben angegebenen Bedeutungen besitzen, in Gegenwart eines zweckmäßigen Lösungsmittels, wie z.B. Ethanol oder Tetrahydrofuran, oder vorzugsweise in Abwesenheit eines Lösungsmittels bei einer Temperatur von 20 bis 100°C zur Umsetzung gebracht wird.

9. Verfahren nach Anspruch 8, bei welchem das Epoxid (II) hergestellt wird durch Behandeln eines Aldehyds der allgemeinen Formel (IV)

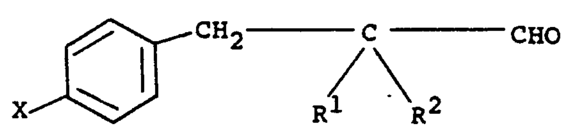

(IV)

mit Dimethyloxosulfoniummethylid oder Dimethylsulfoniummethylid.

10. Fungizide Zusammensetzung, welche eine Verbindung der allgemeinen Formel (I)

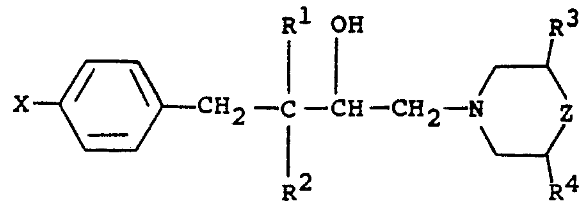

(I)

worin $R^1$, $R^2$, $R^3$ und $R^4$ jeweils für ein Wasserstoffatom oder eine Alkylgruppe mit 1 bis 4 Kohlenstoffatomen stehen, X für ein Wasserstoff- oder Halogenatom oder ein $C_{1-6}$-Alkykl-, Cyclopropyl-, Cyclobutyl, Cyclopentyl-, Cyclohexyl, Alkenyl (mit bis zu Kohlenstoffatomen), Alkinyl- (mit bis zu 6 Kohlenstoffatomen) oder $C_{1-6}$-Alkoxygruppe steht und Z für eine Methylengruppe oder ein Sauerstoffatom steht; oder ein Stereoisomer oder eine Säureadditionssalz davon; oder ein Esterderivat der Alkoholfunktion, das ein Acetyl-, Propionyl- oder Benzoylderivat ist; oder ein Etherderivat der Alkoholfunktion, das ein Methyl-, Ethyl-, Propyl-, Butyl- oder Benzylderivat ist; und ein Träger- oder Verdünnungsmittel enthält.

11. Verfahren zur Bekämpfung von Pilzerkrankungen an einer Pflanze, bei welchem Verfahren auf die Pflanze, auf den Samen der Pflanze oder auf de Standort der Pflanze oder des Samens eine Verbindung der allgemeinen Formel (I)

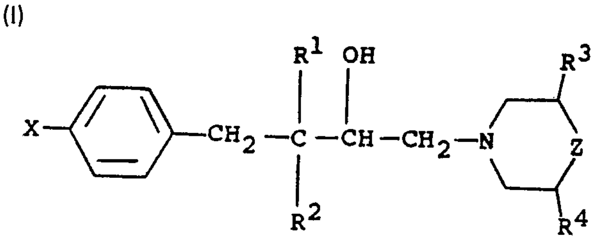

(I)

worin $R^1$, $R^2$, $R^3$ und $R^4$ jeweils fu$C_{1-6}$-r ein Wasserstoffatom oder eine Alkylgruppe mit 1 bis 4 Kohlenstoffatomen stehen, X für ein Wasserstoff- oder Halogenatom oder eine $C_{1-6}$-Alkyl-, Cyclopropyl-, Cyclobutyl-, Cyclopentyl-, Cyclohexyl-, Alkenyl- (mit bis zu 6 Kohlenstoffatomen), Alkinyl- (mit bis zu 6 Kohlenstoffatomen) oder $C_{1-6}$-Alkoxygruppe steht und Z für eine Methylengruppe oder ein Sauerstoffatom steht; oder ein Stereoisomer oder eine Säureadditionssalz davon; oder ein Esterderivat der Alkoholfunktion, das ein Acetyl-, Propionyl- oder Benzoylderivat ist; oder ein Etherderivat der Alkoholfunktion, das ein Methyl-, Ethyl-, Propyl-, Butyl- oder Benzylderivat ist; oder eine Zusammensetzung nach Anspruch 10 aufgebracht wird.

**Revendications**

1. Composé de formule générale (I):

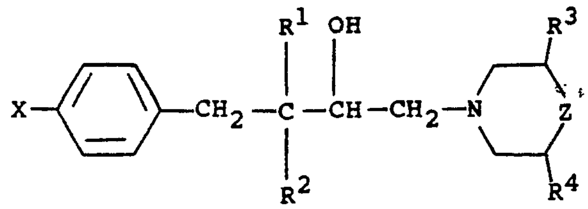

(I)

et ses stéréo-isomères et sels d'addition d'acides, dans lequel $R^1$, $R^2$, $R^3$ et $R^4$ représentent chacun un atome d'hydrogène ou un groupe alkyle contenant 1 à 4 atomes de carbone; X représente un atome d'hydrogène ou un atome d'halogène, ou bien un groupe alkyle en $C_1$ à $C_6$, cyclopropyle, cyclobutyle, cyclopentyle, cyclohexyle, alcényle contenant jusqu'à 6 atomes de carbone, alcynyle contenant jusqu'à 6 atomes de carbone ou alkoxy en $C_1$ à $C_6$; et Z représente un groupe méthylène ou un atome d'oxygène; et des esters dérivés de la fonction alcool qui sont des dérivés d'acétyle, de propionyle ou de benzoyle; et des éthers dérivés de la fonction alcool qui sont des éthers méthyliques (sauf lorsque X représente l'hydrogène ou le chlore et $R^1$, $R^2$, $R^3$ et $R^4$ représentent tous l'hydrogène), éthyliques, propyliques, butyliques ou benzyliques; et leurs sels d'addition d'acides.

2. Composé suivant la revendication 1, dans lequel $R^1$, $R^2$, $R^3$ et $R^4$ représentent chacun l'hydrogène ou un groupe méthyle.

3. Composé suivant la revendication 1 ou la revendication 2, dans lequel X représente l'hydrogène, le chlore ou le flour ou bien un groupe méthyle, éthyle, propyle (*n-* ou *iso*propyle) ou butyle (*n-, sec.-, iso-* ou *tertio*-buyle).

4. Composé suivant l'une quelconque des revendications 1 à 3, dans lequel Z représente un groupe $-CH_2-$.

5. 1-[4-(para-tertio-butylphényl)-3-méthyl-2-hydroxybutyl]-3,5-diméthylpipéridine.

6. 1-[4-(para-tertio-butylphényl)-3-méthyl-2-hhdroxybutyl]pipéridine.

7. Composé suivant la revendication 6, sous forme de son isomère ayant un point de fusion de 67—68°C.

8. Procédé de préparation des composés suivant la revendication 1 et répondant à la formule (I):

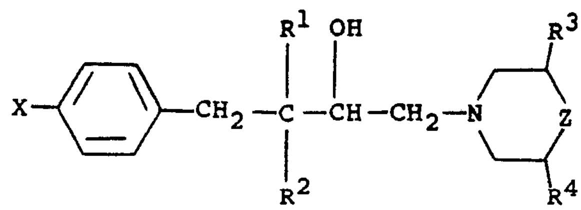

(I)

dans lequel $R^1$, $R^2$, $R^3$ et $R^4$ représentent chacun un atome d'hydrogène ou un groupe alkyle contenant 1 à 4 atomes de carbone; X représente un atome d'hydrogène ou d'halogène, ou bien un groupe alkyle en $C_1$ à $C_6$, cyclopropyle, cyclobutyle, cyclopentyle, cyclohexyle, alcényle contenant jusqu'à 6 atomes de carbone, alcynyle contenant jusqu'à 6 atomes de carbone ou alkoxy en $C_1$ à $C_6$; et Z représente un groupe méthylène ou un atome d'oxygène, qui consiste à mettre en réaction un époxyde de formule générale:

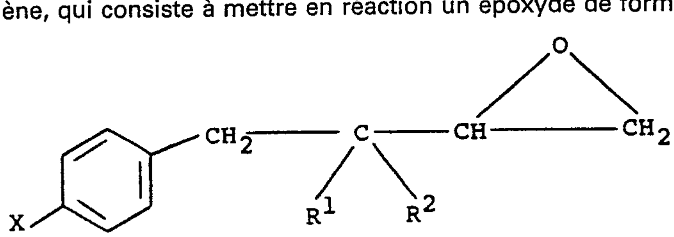

(II)

dans laquelle X, $R^1$ et $R^2$ répondant aux définitions précitées, avec un composé de formule générale (II):

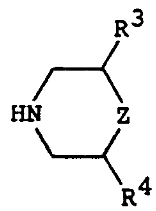

(III)

dans laquelle $R^3$ et $R^4$ et Z répondent aux définitions précitées, en présence d'un solvant convenable tel que l'éthanol ou le tétrahydrofuranne ou bien de préférence, en l'absence d'un solvant à une température de 20 à 100°C.

9. Procédé suivant la revendication 8, dans lequel l'époxyde (II) est préparé par traitement d'un aldéhyde de formule générale (IV):

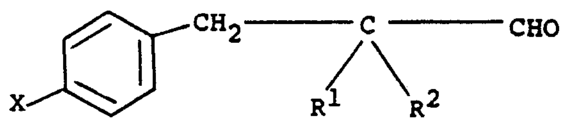

(IV)

avec le méthylure de diméthloxosulfonium ou le méthylure de diméthylsulfonium.

10. Composition fongicide comprenant un composé de formule générale (I):

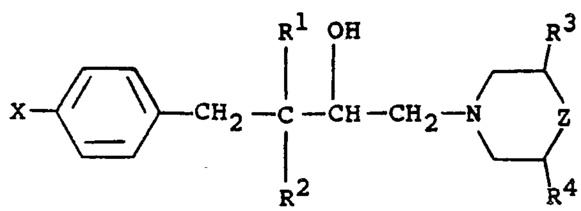

(I)

dans laquelle $R^1$, $R^2$, $R^3$ et $R^4$ représentent chacun un atome d'hydrogène ou un groupe alkyle contenant 1 à 4 atomes de carbone; X représente un atome d'hydrogène ou d'halogène ou bien un groupe alkyle en $C_1$ à $C_6$, cyclopropyle, cyclobutyle, cyclopentyle, cyclohexyle, alcényle contenant jusqu'à 6 atomes de carbone, alcynyle contenant jusqu'à 6 atomes de carbone ou alkoxy en $C_1$ à $C_6$; et Z représente un groupe méthylène ou un atome d'oxygène, ou un de ses stéréo-isomères ou sels d'addition d'acides; ou un ester dérivé de la fonction alcool qui est un dérivé d'acétyle, de propionyle ou de benzoyle; ou un éther dérivé de la fonction alcool qui est un éther méthylique, éthylique, propylique, butylique ou benzylique; et un support ou diluant.

11. Procédé de lutte contre des maladies fongiques chez une plante, qui consiste à appliquer à la plante, aux semences de la plante ou au milieu de la plante ou des semences, un composé de formule générale (I):

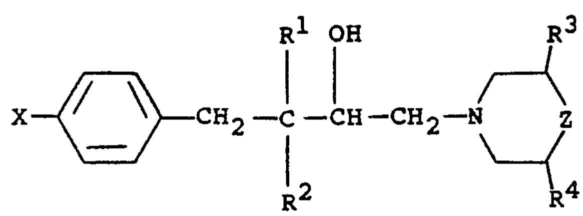

(I)

dans laquelle $R^1$, $R^2$, $R^3$ et $R^4$ représentent chacun un atome d'hydrogène ou un groupe alkyle contenant 1 à 4 atomes de carbone; X représente un atome d'hydrogène ou atome d'halogène ou bien un groupe alkyle en $C_1$ à $C_6$, cyclopropyle, cyclobutyle, cyclopentyle ou cyclohexyle, alcényle contenant jusqu'à 6 atomes de carbone, alcynyle contenant jusqu'à 6 atomes de carbone ou alkoxy en $C_1$ à $C_6$; et Z représente un groupe méthylène ou un atome d'oxygène; ou un de ses stéréo-isomères ou sels d'addition d'acides; ou un ester dérivé de la fonction alcool qui est un dérivé d'acétyle, de propionyle ou de benzoyle; ou un éther dérivé de la fonction alcool qui est un éther méthylique, éthylique, propylique, butylique ou benzylique; ou bien une composition suivant la revendication 10.